# EUROPEAN PATENT SPECIFICATION

(11) **EP 2 482 783 B1**
(45) Date of publication and mention of the grant of the patent: **29.07.2015**
(21) Application number: 10768268.4
(22) Date of filing: 01.10.2010
(51) Int. Cl.: A61G 9/00

(54) **A moulded pulp urinal**
Urinbecken aus einem Fasergussteil
Urinal en pâte cellulosique moulée

(30) Priority: 01.10.2009 GB 0917207
(43) Date of publication of application: 08.08.2012
(73) Proprietor: HPC Healthline Limited, Morden, Surrey SM4 4LZ (GB)
(72) Inventor: KHAN, Fuad ul-Haque, Pontllanfraith Gwent NP12 2AN (GB); KIRK, Harry Scott, Pontllanfraith Gwent NP12 2AN (GB); KHAN, Mohammed Jalaluddin, Pontllanfraith Gwent NP12 2AN (GB); ROSE, Harvey Leslie, Peterborough Cambridgeshire PE2 6YQ (GB)
(74) Representative: Jones, David Alan
(86) International application number: PCT/GB2010/001845
(87) International publication number: WO 2011/039516

(56) References cited:
- GB-A- 2 019 209
- GB-A- 2 059 257
- US-A- 2 742 218
- US-A- 3 579 653
- US-A- 6 026 519

## Description

The present invention relates to urinals, and in particular to disposable urinals made from moulded pulp fibre.

Conventional urinals for use in hospitals by patients in bed traditionally comprise a body portion defining a substantially enclosed container with an extended spout like portion leading to an opening. Such urinals are intended to be reusable and are constructed from metal or other rigid plastic materials. After use, these urinals have to be emptied, cleaned and sterilised. This process can be problematic, due in part to the shape of the urinal, and the re-use of such urinals can lead to the risk of cross contamination.

More recently, disposable urinals of a similar shape and configuration to conventional reusable urinals have been adopted. Such disposable urinals comprise a semi rigid one piece moulded receptacle made from for example moulded pulp fibres. These disposable urinals are simply disposed of after use, for example in macerating machines. This avoids the need for emptying, cleaning and sterilisation of the urinal. However, a problem with such urinals is that they can be difficult to produce and in general require complex moulding machinery due to their hollow shape and small narrow opening and neck which is smaller than the main body. In addition, the hollow enclosed shape means that they take up a relatively large amount of space, including a substantial amount of 'dead-air', therefore presenting a storage problem and increasing shipping costs. For the same reason conventional reusable urinals are also difficult to store and ship.

To address these further problems it has been proposed, for example in GB 2019209, to produce a disposable moulded fibre urinal from two separate and separately moulded portions which are subsequently secured together. Such an arrangement, as described, is easier to manufacture, and requires less storage space and transfer costs if the two halves are put together at the point of use. However in order to prevent leakage between and along the seam and join line of the two halves the two halves must be carefully aligned and sealed together prior. This operation is difficult with the arrangement of GB'209, which requires the two parts to be aligned by eye and secured at the point of use. Accordingly, and more generally this arrangement can be further improved.

It has also been previously proposed in GB 596930 to produce a reusable urinal having an outer casing formed from two metal hinged sections into which is located a removable disposable liner. The casing itself is not watertight and the liner is required to contain the liquid and prevent leakage between the two hinged sections split along the midline of the vessel body. Following use the liner can be disposed of together with the contents, thereby making emptying and cleaning of the hinged casing easier. The addition of a removable liner however adds complexity and the removable outer sections of the vessel still need to be cleaned and sterilised before reuse.

Urinals may be for male use, or more recently also for female use as for example described in WO2007/1 19076. In addition it is also known to produce other disposable products from moulded pulp for both clinical and other uses.

US 3,579,653 describes a flat pack foldable urinal which is able to be stored as a flat sheet but which requires complex assembly at the point of use.

It is however desirable to provide an improved disposable moulded pulp urinal which addresses the above described problems and/or which more generally offers improvements or an alternative to existing arrangements.

According to the present invention there is therefore provided a disposable urinal as described in the accompanying claims.

In an embodiment of the invention there is provided a disposable urinal comprising first and second mating sections each including a mating surface, the mating surfaces being configured to abut and mate against each other, and means for securing the mating surfaces of the first and second sections together when they are abutted, the urinal further comprising a hinge interconnecting the first and second sections together such that they can be pivoted about the hinge from a disassembled position to an assembled position in which the mating surfaces of the first and second mating sections abut.

Such a two section disposable urinal is easier to manufacture, store and ship in the disassembled configuration. Furthermore, the hinge ensures that the two sections are retained together and that the mating surfaces are accurately aligned when moved into abutment in the assembled configuration. In this way, an improved securement and sealing is provided between the first and second sections which prevents leakage from the container and ensures that the two sections are held together in use.

The first and second sections are preferably disposed end to end longitudinally adjacent to each other in the disassembled position. When in the disassembled position the first and second sections are preferably nestable within respective first and second sections of a second urinal when in the disassembled position. This arrangement simplifies manufacture, enabling simplified mould structure and narrower arrangement of the mould components along a manufacturing line. In addition, providing the hinge at the rear end of the first and second sections enables the securing means to be provided along the length of both longitudinal mating surfaces.

The second section preferably overlies the first section in the assembled position.

The first section comprises a bottom section, and the second section comprises a top section configured to mate on top of the bottom section. The bottom section comprises a container having a base and side walls extending from said base and supporting said mating flange spaced from said base, and the top section comprises a lid to the container.

The second section may include and define at least in part an opening to the urinal. The first section may also include and defines part of said opening to the urinal.

The second section may include an arched cowl portion. Preferably the arched cowl portion extends from and defining at least in part said opening.

At least one of the first or section sections may further comprise at least one projection which locates within a recess defined in the other section when the first and second section are abutted in the assembled position. In particular the second section may include at least one projection which locates within at least part of said opening when the first and second sections are abutted in the assembled position.

Preferably the means for securing comprises a means for adhering the flanges together. The means for adhering comprises an adhesive layer applied to at least one of the first and second mating surfaces configured to secure the mating surfaces together and create a seal therebetween.

The adhesive layer may include a release sheet and having a base surface secured to the at least one of the first and second mating surfaces and a top surface to which the release sheet is removabley secured. The adhesive may therefore be pre-applied to the mating surfaces at the precise locations required. The release strip obscures the adhesive layer and prevents its upper surface from curing and/or from adhering to the second section of other urinals during transit. The release sheet is then removable by the user at the point of use, and the first and second sections adhered together without requiring complicated assembly or the application of an adhesive by the user. The pre-application of the adhesive also ensures that sealing and securing between the first and second sections is achieved at the precise locations required.

Preferably the first and second sections and hinge interconnecting them are integral and comprise a unitary structure. As such, the hinge is formed as a living hinge between the first and second sections. The urinal is furthermore preferably formed from moulded fibre pulp.

The present invention will now be described by way of example only with reference to the following figures in which:
Figure 1 is a schematic illustration of the assembled urinal in accordance with an embodiment of the invention;
Figure 2 is a perspective schematic illustration of the urinal shown in Figure 1 but in the unassembled, open, configuration;
Figure 3 is an end view of the assembled urinal shown in Figure 1;
Figure 4 is a side view of the assembled urinal shown in Figure 1;
Figure 5 is an end view of the unassembled, open, urinal shown in Figure 2;
Figure 6 is a top view of the unassembled, open, urinal shown in Figure 2;
Figure 7 is a perspective schematic illustration of an assembled urinal according to a second embodiment of the invention;
Figure 8 is a underside perspective view of the urinal shown in

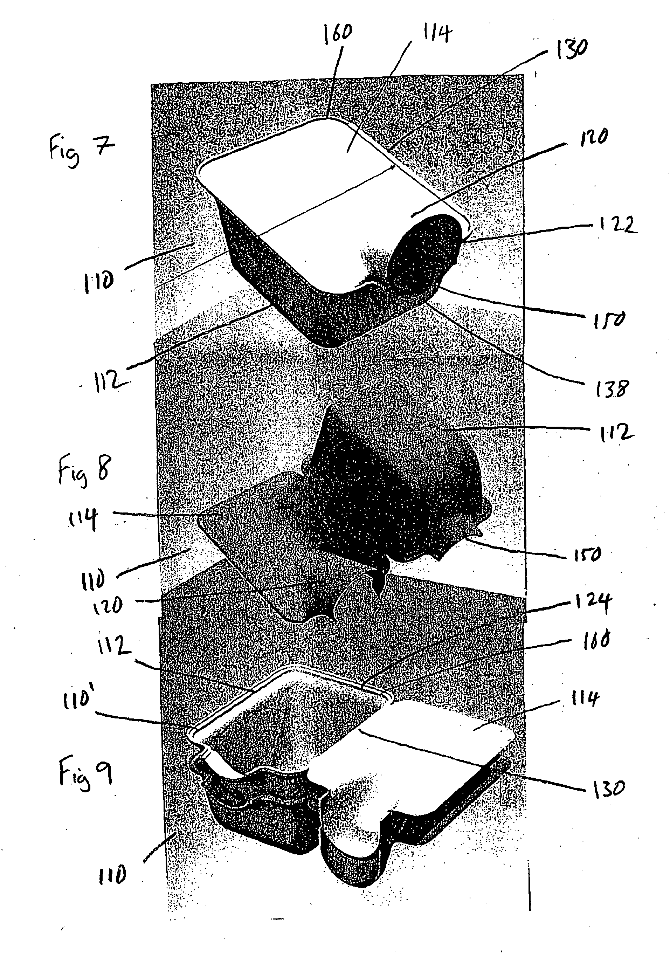

Figure 7 but in a unassembled, open configuration; and
Figure 9 is an isometric view of two of the urinals shown in Figure in a disassembled configuration and stacked on top of each other.

In the following description of the invention, certain terminology will be used for the purpose of reference only, and is not intended to be limiting. Terms such as "upper", "lower", "above", "below", "rightward", "leftward", "clockwise", and "counterclockwise" refer to directions in the drawings to which reference is made. Terms such as "inward" and "outward" refer to directions toward and away from, respectively, the geometric centre of the component described. Terms such as "front", "rear", "side", "leftside", "rightside", "top", "bottom", "horizontal", and "vertical" describe the orientation of portions of the component within a consistent but arbitrary frame of reference which is made clear by reference to the text and the associated drawings describing the component under discussion. The term "urinal" refers to a liquid vessel having a body section defining a liquid receptacle, and an opening for permitting the ingress of liquid into the receptacle. Such terminology will include the words specifically mentioned above, derivatives thereof, and words of similar import.

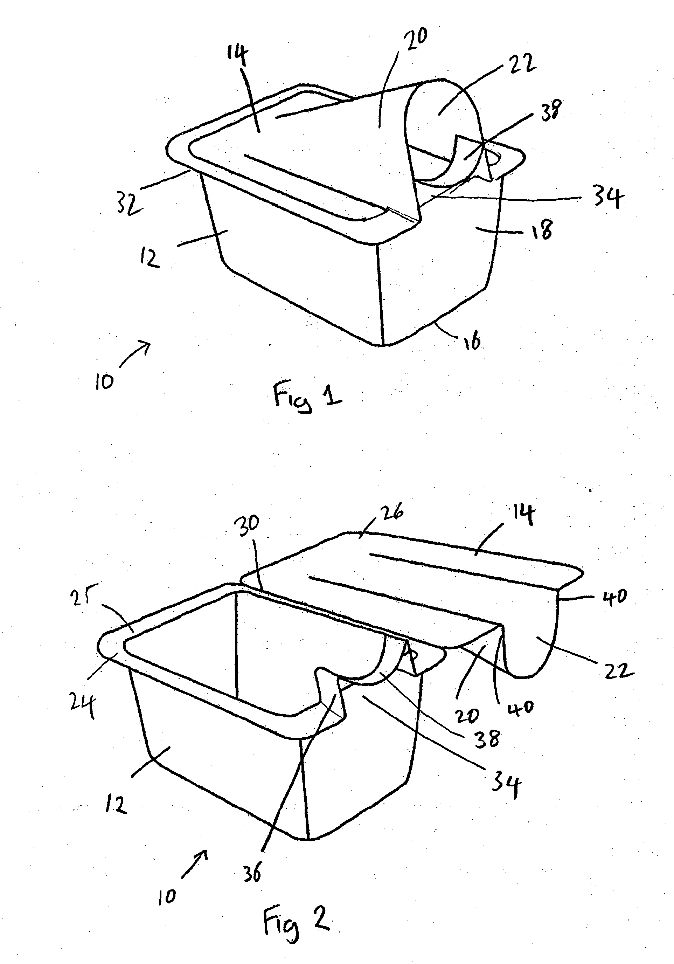

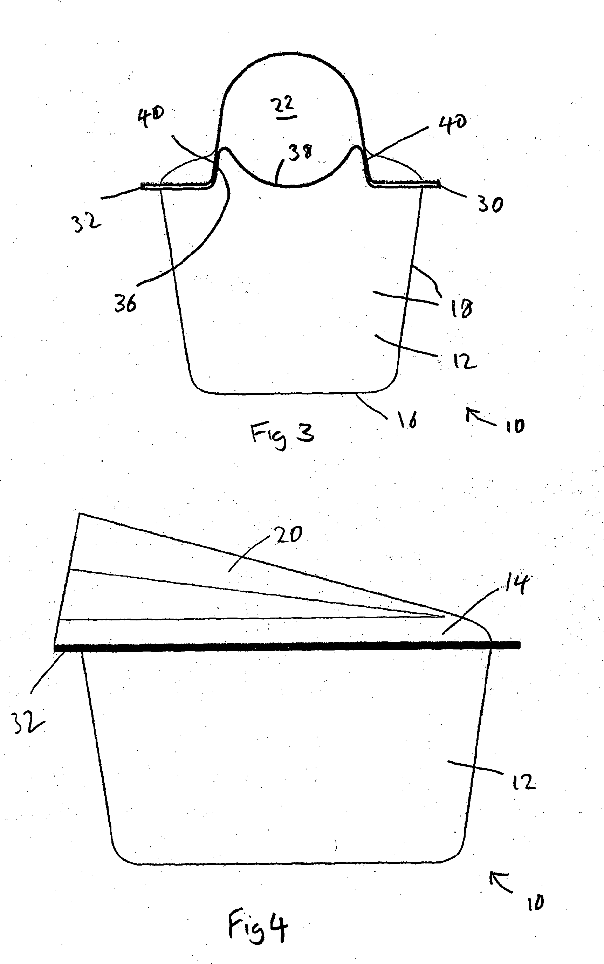

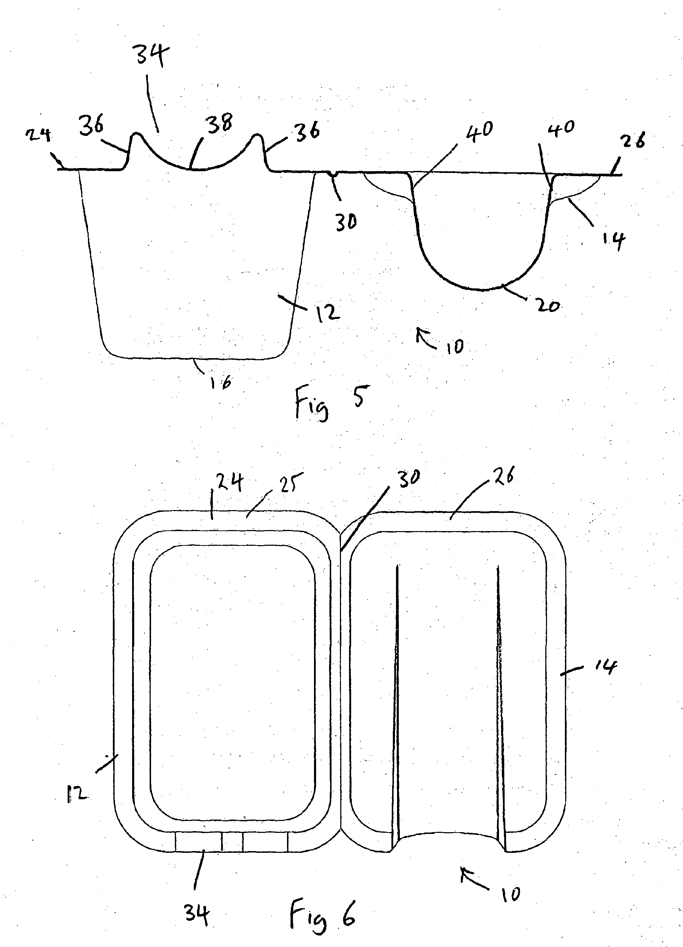

Referring to Figures 1 to 6, a urinal 10 in accordance with an embodiment of the invention comprises a bottom or main body section 12 and an upper or lid or top section 14, which mate with one another to form the assembled urinal 10 as shown in Figures 1, 3 and 4. The bottom 12 and top 14 sections are pre-formed substantially rigid elements. The bottom or main body section 12 comprises a base 16 and upstanding walls 18 defining an open topped container. The top section or lid 14 consists of a generally planar structure with an arched tunnel or cowl portion 20 comprising and defining an arched opening 22 and tapering down towards the flat remainder of the lid section 14. The top and bottom sections 14, 12 each include corresponding mating surfaces defined by peripheral flanges 24 and 26 extending substantially all around the edges of each of the top and bottom sections 14, 12, except in the region of the opening 22.

The assembled urinal 10 as shown in Figures 1, 3, and 4 is generally similar to a conventional urinal having an opening or mouth 22 and cowl section 20 forming a spout to receive urine which is then collected and retained in the main body section 12, with the top section 14 enclosing a urinal. In this particular embodiment the urinal is generally rectangular although it is appreciated that in other arrangements the urinal 10 may have other shapes. Preferably the opening 22 is defined by portions of both the top 14 and bottom 12 sections. In an alternative arrangement, the opening to the urinal 10 may be defined by an aperture formed in one of the top 14 and bottom 12 sections, with a spout optionally being provided.

The urinal 10 and top 14 and bottom 12 sections are moulded from pulp fibre material. Such moulding itself is well known and will not be described in detail. Pulp material is particularly beneficial for this application as it enables the urinal 10 to be disposable due to its cost being such that it is less expensive to manufacture a new one than to clean and recycle a re-usable urinal (for example formed of metal of dense plastics) to make it fit for use again. In addition, pulp moulded products may be easily broken down, for example by maceration, and are biodegradable, thereby providing improved environmental benefits. Alternatively, the urinal 10 may be formed of a disposable plastics material, and preferably a biodegradable plastic.

The top 14 and bottom 12 sections are hingedly connected together along, in this case, one side, which may include one end, by a living hinge 30 and are preferably integral and formed as a single unitary structure. While the top 14 and bottom 12 sections are shown in the Figures as hingedly connected along adjacent sides, it will however be appreciated that in other arrangements the hinged 30 and top and bottom sections 12, 14 may be connected together along the end edges depending on the shape of the urinal 10, such that in the disassembled condition the top 14 and bottom 12 sections are longitudinally adjacent. Also, in other embodiments the top 14 and bottom sections 12 may be separately formed and then joined together by a separate hinge 30 or tape. This however is less preferred.

The urinal 10 may be stored and shipped in the disassembled configuration as shown in Figures 2, 5 and 6 with the urinals 10 nested and stacked on top of one another as shown for example in relation to the second embodiment shown in Figure 9. In order to enable the urinals 10 to be so nested and stacked the top 14 and bottom 12 sections and in particular walls 18 and cowl 20 are inwardly tapered.

One or both of the flanges 24 and/or 26 defining the mating surfaces is provided with a layer of adhesive 25 for securing the flanges 24 and 26 and so top 14 and bottom 12 sections together to form a seal between the two sections 12 and 14. The layer of adhesive may be applied to discrete sections of the corresponding flange 24 and/or 26, or may define a band of adhesive tape 25 extending along the entire length of the flange 24 or 26, such that it extends around substantially the entire periphery of the top 14 and/or bottom 12 sections.

The adhesive layer 25 is provided with a removable release sheet, with the base surface of the adhesive layer being adhered to the respective flange 24 and/or 26, and the top surface having the release sheet applied thereto. The removable release sheet covers the adhesive 25 when the urinal 10 is in the disassembled state with this release backing being removed immediately prior to assembly. The adhesive 25 is preferably provided in the form of a pressure sensitive adhesive. The adhesive 25 may be provided in tape format and may single or double sided.

Alternatively in other arrangements a pressure sensitive adhesive or gel may be directly applied to one or both the flange surfaces 24, 26 and/or other suitable arrangements provided to adhere the two flanges 24, 26 and sections 12, 14 together. Where the adhesive layer 25 is applied directly a removable release strip is then applied in a subsequent step to prevent it adhering in the disassembled state, and which is then removed when the urinal 10 is to be assembled prior to use. In preferred arrangements the adhesive tape 25, and/or adhesive is such that it bonds to the relatively rough moulded pulp material, forms a suitable seal. The adhesive 25 is water soluble and biodegradable which ensures that it will break down with the pulp when the urinal 10 is macerated after use and in the waste system without causing clumping of the macerated pulp. It should also be noted that when assembled, the top 14 and bottom 12 sections are joined along a seam 32, this seam 32 is substantially spaced above the base 16 of the base section 12 and urinal 10, and is well above the general in use level of any liquid within the urinal. As such the seam 32, and so adhesive seal is not generally subjected to the liquid and therefore the adhesive does not necessarily need to provide a complete seal and be completely water tight (although this is desirable and beneficial), and primarily simply needs to hold the two sections 12,14 of the urinal 10 together in the assembled condition.

The flanges 24, 26 may include securing means defined by interlocking features moulded therein to hold and interlock the flanges 24, 26 and so sections together. Such interlocking features may be in place of or in addition to any adhesive tape 25 or glue. Other means for securing and/or adhering the flanges 24, 26 and sections 12, 14 together could also be used. Alternatively or in addition, the flanges 24, 26 may include a securing means defined by a tongue and groove arrangement in which one of the flanges 24, 26 includes a groove or channel defined along at least part of its length, and the other flange 24, 26 includes a corresponding ridge or tongue extending therefrom at a corresponding position along its length. The groove is configured to received and hold the ridge portion to secure the top section 14 to the bottom section 12. It will be appreciated that the term flange refers to the mating surface defined at the upper edges of the top 14 and bottom 12 sections at the distal edge of their upstanding walls. While it is preferable the flanges 24, 26 project laterally outwards from the walls to which they are connected this is not essential and the flanges may be of the same width as the walls providing this width provides a suitably sized mating surface sufficient to achieve suitable mating and sealing between the top 14 and bottom 12 sections.

To form and assemble the urinal 10 the release sheet of the adhesive layer is firstly removed to expose the adhesive. The top section 14 is then moved about the hinge 30 to the assembled position such that it lies on top of the bottom section 12 with the mating flanges 26 and 24 then abutting. As the top and bottom sections 12, 14 are connected together by a living hinge 30 in the disassembled condition (as shown in Figures 2, 5 and 6), the two parts of the urinal 10 are kept together so they are not lost. As the top section 14 is moved to the assembled position, the hinge 30 ensures that the flanges 24 and 26 are correctly aligned when the top 14 is folded over on top of the bottom section 12 ensuring that they accurately align and mate with each other to optimise the adhesion and sealing between the two mating surfaces. Pressure is applied by the user to urge the flanges 24 and 26 against each other to promote adhesion. This is particularly important where a pressure sensitive adhesive is used.

The bottom section 12 also preferably includes a projecting portion 34 which projects upwardly above flange 24 of the bottom section 12 in the region of the mouth opening 22 of the lid section 14 when the urinal 10 is assembled. The projecting section 34 in particular is configured to fit within the bottom of the arched mouth opening 22 of the cowl 20 of the top lid section 14 and includes side flanges 35 which abut against bottom sections 40 of the side walls of the cowl 20. The projection 34 also includes a further curved lip flange 38 which defines a bottom rim of the mouth opening 22. The flanged lip 38 provides a smooth bottom section to the mouth opening 22 for receiving a male organ when the urinal is in use. The projecting portion 34 and its engagement within the arched cowl 20 of the lid 14 reinforces and strengthens the cowl 20 and lid 14 as well as ensuring that the top lid section 14 is correctly located and aligned on the bottom section 12. In particular the location of the projecting portion 34 within the cowl 20 and lid 14 centres the lid 14 on top of the bottom section 12 such that the peripheral flanges 24 26 are aligned when the urinal 10 is assembled. It will be appreciated that in other embodiments further projections (not shown) from the bottom section 12 and which engage in further recesses defined in the top lid section 14 could be provided, and also that the arrangement could be reversed with all or some of the projections and projecting portions 34 projecting from the top lid 14 and engaging corresponding recesses in the bottom section 12 to further or alternatively align and reinforce the location of the top section 12 on the bottom section 14.

Referring to Figures 7 to 9, a urinal 100 according to another embodiment of the invention is shown. This urinal 110 is generally similar to the urinal 10 shown in Figures 1 to 6 and like reference numerals, incremented by 100 will be used to indicate corresponding features. Only the main difference between this urinal 110 and urinal 10 of the previous embodiment will be described. In particular in this embodiment the urinal 110 does not include the projecting portion 34 but instead includes a curved arcuate scalloped section 150 in the top flange 124 of the bottom section 112 in the region of the opening 122 and which defines a lower lip surface 138 of the opening 122. This embodiment in the flange 124 of the bottom section 122 also includes a peripheral upstand 160 around the flange 124 defining a rim surrounding the peripheral edge of the top section 114 when the top section 114 is folded on top of the bottom section 122 as shown in figure 7. This upstand 160 also preferably includes a lip (not shown) which projects inwardly over the top of the top portion 114 when this is folded on top of the bottom section 112 to thereby hold an edge of the top section 114 onto the bottom section 112 and also locate the edge.

It will be appreciated that there may be further modifications to the arrangement described above and in particular the features of the two separately described embodiments may be combined and mixed in other embodiments. In particular, as indicated above the exact shape of the urinal may be changed and in particular the urinal may be more rounded comprising for example a lipoid shape. In addition while as shown the urinal of these embodiment is primarily for male use it is also known to produce a female urinal or other similar products.

The principle and mode of operation of this invention have been explained and illustrated in its preferred embodiment. However, it must be understood that this invention may be practiced otherwise than as specifically explained and illustrated without departing from its scope as defined by the appended claims.

## Claims

1. A disposable urinal comprising:
First (12) and second (14) mating sections each including a mating surface (24,26), the mating surfaces (24,26) being configured to abut and mate against each other;
means for securing the mating surfaces (24,26) of the first (12) and second (14) sections together when they are abutted; and
a hinge (30) interconnecting the first (12) and second (14) sections together such that they can be pivoted about the hinge (30) from a disassembled position to an assembled position in which the mating surfaces (24,26) of the first (12) and second (14) mating sections abut;
**characterised in that**:
the first section (12) comprises a bottom section including a base (16) and side walls (18) defining a container with the side walls (18) extending from said base (16) and supporting said mating surface (24) spaced from said base (16), and the second section (14) comprises a top section forming a lid for the container configured to mate on top of the bottom section (12).

2. A disposable urinal as defined in claim 1 in which the second section (14) includes and defines at least in part an opening (22) to the urinal.

3. A disposable urinal as defined in claim 2 in which the first section (12) includes and defines at least part of said opening (22) to the urinal.

4. A disposable urinal as defined in any preceding claim in which the second section (14) includes an arched cowl portion (20).

5. A disposable urinal as defined in claim 2 or 3 in which the second section (14)includes an arched cowl portion (20) extending from and defining at least part said opening (22).

6. A disposable urinal as defined in any preceding claim in which one of the first (12) or second (14) sections further comprises at least one projection which locates within a recess defined in the other section when the first (12) and second (14) section are abutted in the assembled position.

7. A disposable urinal as defined in claim 2 or 3 or 5 in which the second section (14) further comprises at least one projection which locates within at least part of said opening when the first (12) and second (14) section are abutted in the assembled position.

8. A disposable urinal as defined in any preceding claim wherein the means for securing comprises a means for adhering and sealing the mating surfaces (24,26) together.

9. A disposable urinal as defined in claim 8 wherein the means for adhering comprises an adhesive layer applied to at least one of the first and second mating surfaces (24,26), the adhesive layer including a release sheet and having a base surface secured to the at least one of the first and second mating surfaces (24,26) and a top surface to which the release sheet is removabley secured.

10. A disposable urinal as defined in any preceding claim in which the first and second sections and hinge interconnecting them are integral and comprise a unitary structure.

11. A disposable urinal as defined in any preceding claim wherein the first (12) and second (14) sections are pre-formed substantially rigid elements.

12. A disposable urinal as defined in any preceding claim wherein the first (12) and second (14) section are moulded elements moulded from pulp fibre.

13. A method of manufacturing a disposable urinal comprising forming first (12) and second (14) mating sections each including a mating surface (24,26), the mating surfaces being configured to abut and mate against each other; means for securing the mating surfaces (24,26) of the first (12) and second (14) sections together when they are abutted; and
a hinge (30) interconnecting the first (12) and second (14) sections together such that they can be pivoted about the hinge (30) from a disassembled position to an assembled position in which the mating surfaces (24,26) of the first (12) and second (14) mating sections abut; wherein the first section (12) comprises a base (16) and side walls (18) defining a container with the side walls (18) extending from said base (16) and supporting said mating surface spaced from said base (16), and the second section (14) comprises a lid for the container.

14. A method of manufacturing a disposable urinal as defined in claim 13 further comprising providing an adhesive layer on at least one of the mating surfaces (24,26) of the first (12) and second (14) mating sections, the adhesive layer being configured to secure the mating surface together and comprising a release sheet for removal at the point of use.

## Patentansprüche

1. Einwegurinal, umfassend:
einen ersten (12) und einen zweiten (14) Passabschnitt, die jeweils eine Passfläche (24, 26) aufweisen, wobei die Passflächen (24, 26) dazu konfiguriert sind, aneinander anzustoßen und zusammenzupassen,
ein Mittel zum Befestigen der Passflächen (24, 26) des ersten (12) und des zweiten Abschnitts (14) aneinander, wenn sie aneinander anstoßen, und
ein Scharnier (30), das den ersten (12) und den zweiten (14) Abschnitt miteinander verbindet, so dass sie um das Scharnier (30) aus einer auseinandermontierten Position in eine montierte Position, in der die Passflächen (24, 26) des ersten (12) und des zweiten (14) Passabschnitts zusammengefügt sind, geschwenkt werden können,
**dadurch gekennzeichnet, dass**
der erste Abschnitt (12) einen Bodenabschnitt umfasst, der eine Basis (16) und Seitenwände (18) aufweist, die einen Behälter definieren, wobei sich die Seitenwände (18) von der Basis (16) erstrecken und die von der Basis (16) beabstandete Passfläche (24) stützen, und der zweite Abschnitt (14) einen oberen Abschnitt umfasst, der einen Deckel für den Behälter bildet, der dazu konfiguriert ist, mit der Oberseite des unteren Abschnitts (12) zusammengefügt zu werden.

2. Einwegurinal nach Anspruch 1, wobei der zweite Abschnitt (14) mindestens teilweise eine Öffnung (22) zum Urinal aufweist und definiert.

3. Einwegurinal nach Anspruch 2, wobei der erste Abschnitt (12) mindestens einen Teil der Öffnung (22) zum Urinal aufweist und definiert.

4. Einwegurinal nach einem der vorhergehenden Ansprüche, wobei der zweite Abschnitt (14) einen gebogenen Haubenabschnitt (20) aufweist.

5. Einwegurinal nach Anspruch 2 oder 3, wobei der zweite Abschnitt (14) einen gebogenen Haubenabschnitt (20) aufweist, der sich von mindestens einem Teil der Öffnung (22) erstreckt und diesen definiert.

6. Einwegurinal nach einem der vorhergehenden Ansprüche, wobei der erste (12) oder der zweite (14) Abschnitt ferner mindestens einen Vorsprung umfasst, der in einer im anderen Abschnitt definierten Aussparung angeordnet ist, wenn der erste (12) und der zweite (14) Abschnitt in der montierten Position aneinander anstoßen.

7. Einwegurinal nach Anspruch 2 oder 3 oder 5, wobei der zweite Abschnitt (14) ferner mindestens einen Vorsprung umfasst, der in mindestens einem Teil der Öffnung angeordnet ist, wenn der erste (12) und der zweite (14) Abschnitt in der montierten Position aneinander anstoßen.

8. Einwegurinal nach einem der vorhergehenden Ansprüche, wobei das Befestigungsmittel ein Mittel umfasst, um die Passflächen (24, 26) zusammenzukleben und miteinander zu versiegeln.

9. Einwegurinal nach Anspruch 8, wobei das Klebemittel eine Klebstoffschicht umfasst, die auf mindestens eine der ersten und zweiten Passfläche (24, 26) aufgebracht ist, wobei die Klebstoffschicht eine Trennfolie aufweist und eine Basisfläche, die an der ersten und/oder der zweiten Passfläche (24, 26) befestigt ist, und eine obere Fläche hat, an der die Trennfolie entfernbar befestigt ist.

10. Einwegurinal nach einem der vorhergehenden Ansprüche, wobei der erste und der zweite Abschnitt und das sie verbindende Scharnier integral sind und eine einteilige Struktur umfassen.

11. Einwegurinal nach einem der vorhergehenden Ansprüche, wobei der erste (12) und der zweite (14) Abschnitt vorgeformte, im Wesentlichen starre Elemente sind.

12. Einwegurinal nach einem der vorhergehenden Ansprüche, wobei der erste (12) und der zweite (14) Abschnitt aus Zellstofffaser geformte Formelemente sind.

13. Verfahren zur Herstellung eines Einwegurinals, umfassend das Bilden des ersten (12) und des zweiten (14) Passabschnitts, die jeweils eine Passfläche (24, 26) aufweisen, wobei die Passflächen dazu konfiguriert sind, aneinander anzustoßen und zusammenzupassen, ein Mittel, um die Passflächen (24, 26) des ersten (12) und des zweiten (14) Abschnitts aneinander zu befestigen, wenn sie aneinander anstoßen, und
ein Scharnier (30), das den ersten (12) und den zweiten (14) Abschnitt miteinander verbindet, so dass sie um das Scharnier (30) aus einer auseinandermontierten Position in eine montierte Position, in der die Passflächen (24, 26) des ersten (12) und des zweiten (14) Passabschnitts aneinander anstoßen, geschwenkt werden können, wobei der erste Abschnitt (12) eine Basis (16) und Seitenwände (18) aufweist, die einen Behälter definieren, wobei sich die Seitenwände (18) von der Basis (16) erstrecken und die von der Basis (16) beabstandete Passfläche stützen, und der zweite Abschnitt (14) einen Deckel für den Behälter umfasst.

14. Verfahren zur Herstellung eines Einwegurinals nach Anspruch 13, ferner umfassend das Bereitstellen einer Klebstoffschicht auf mindestens einer der Passflächen (24, 26) des ersten (12) und zweiten (14) Passabschnitts, wobei die Klebstoffschicht dazu konfiguriert ist, die Passflächen aneinander zu befestigen, und eine Trennfolie zur Entfernung am Verwendungsort umfasst.

## Revendications

1. Urinal jetable, comprenant:
des première (12) et deuxième (14) sections de jonction présentant chacune une surface de jonction (24 26), les surfaces de jonction (24, 26) étant configurées pour être mises bout à bout et jointes l'une contre l'autre;
des moyens pour fixer les surfaces de jonction (24, 26) des première (12) et deuxième (14) sections l'une à l'autre lorsqu'elles sont bout à bout; et
une charnière (30) qui interconnecte les première (12) et deuxième (14) sections l'une à l'autre de telle sorte qu'elles puissent pivoter autour de la charnière (30) depuis une position désassemblée jusqu'à une position assemblée dans laquelle les surfaces de jonction (24, 26) des première (12) et deuxième (14) sections sont bout à bout;
**caractérisé en ce que**:
la première section (12) comprend une section inférieure comprenant une base (16) et des parois latérales (18) qui définissent un récipient, les parois latérales (18) s'étendant à partir de ladite base (16) et supportant ladite surface de jonction (24) espacée de ladite base (16), et la deuxième section (14) comprend une section supérieure qui forme un couvercle pour le récipient configuré pour épouser le sommet de la section inférieure (12).

2. Urinal jetable selon la revendication 1, dans lequel la deuxième section (14) comprend et définit au moins en partie une ouverture (22) vers l'urinal.

3. Urinal jetable selon la revendication 2, dans lequel la première section (12) comprend et définit au moins une partie de ladite ouverture (22) vers l'urinal.

4. Urinal jetable selon l'une quelconque des revendications précédentes, dans lequel la deuxième section (14) comprend une partie de capot arquée (20).

5. Urinal jetable selon la revendication 2 ou 3, dans lequel la deuxième partie (14) comprend une partie de capot arquée (20) qui s'étend à partir de et définit au moins en partie ladite ouverture (22).

6. Urinal jetable selon l'une quelconque des revendications précédentes, dans lequel une des première (12) ou deuxième (14) sections comporte en outre au moins une saillie qui se loge à l'intérieur d'un renfoncement défini dans l'autre section lorsque les première (12) et deuxième (14) sections sont mises bout à bout dans la position assemblée.

7. Urinal jetable selon la revendication 2 ou 3 ou 5, dans lequel la deuxième section (14) comporte en outre au moins une saillie qui se loge à l'intérieur d'au moins une partie de ladite ouverture lorsque les première (12) et deuxième (14) sections sont mises bout à bout dans la position assemblée.

8. Urinal jetable selon l'une quelconque des revendications précédentes, dans lequel les moyens de fixation comprennent des moyens pour coller et sceller les surfaces de jonction (24, 26) l'une à l'autre.

9. Urinal jetable selon la revendication 8, dans lequel les moyens de collage comprennent une couche adhésive qui est appliquée à au moins une des première et deuxième surfaces de jonction (24, 26), la couche adhésive comprenant un film détachable et présentant une surface de base fixée à ladite au moins une des première et deuxième surfaces de jonction (24, 26), et une surface supérieure à laquelle le film détachable est fixé de façon amovible.

10. Urinal jetable selon l'une quelconque des revendications précédentes, dans lequel les première et deuxième sections et la charnière qui les interconnecte sont intégrées et présentent une structure unitaire.

11. Urinal jetable selon l'une quelconque des revendications précédentes, dans lequel les première (12) et deuxième (14) sections sont des éléments sensiblement rigides préformés.

12. Urinal jetable selon l'une quelconque des revendications précédentes, dans lequel les première (12) et deuxième (14) sections sont des éléments moulés à partir de fibre de pâte cellulosique.

13. Procédé de fabrication d'un urinal jetable, comprenant la formation d'une première (12) et d'une deuxième (14) sections de jonction présentant chacune une surface de jonction (24, 26), les surfaces de jonction étant configurées pour être mises bout à bout et se joindre l'une contre l'autre; des moyens pour fixer les surfaces de jonction (24, 26) des première (12) et deuxième (14) sections l'une à l'autre lorsqu'elles sont mises bout à bout; et
une charnière (30) qui interconnecte la première (12) et la deuxième (14) sections l'une à l'autre de telle sorte qu'elles puissent pivoter autour de la charnière (30) depuis une position désassemblée jusqu'à une position assemblée dans laquelle les surfaces de jonction (24, 26) des première (12) et deuxième (14) surfaces de jonction sont bout à bout; dans lequel la première section (12) comprend une base (16) et des parois latérales (18) qui définissent un récipient, les parois latérales (18) s'étendant à partir de ladite base (16) et supportant ladite surface de jonction espacée de ladite base (16), et la deuxième section (14) comprend un couvercle pour le récipient.

14. Procédé de fabrication d'un urinal jetable selon la revendication 13, comprenant en outre l'application d'une couche adhésive sur au moins une des surfaces de jonction (24, 26) des première (12) et deuxième (14) sections de jonction, la couche adhésive étant configurée de manière à fixer les surfaces de jonction l'une à l'autre et comprenant un film détachable à enlever au moment de l'utilisation.
